(19) Europäisches Patentamt
European Patent Office
Office européen des brevets

(11) **EP 4 699 650 A1**

(12) **EUROPEAN PATENT APPLICATION**

(43) Date of publication:
**25.02.2026 Bulletin 2026/09**

(21) Application number: **25196776.6**

(22) Date of filing: **19.08.2025**

(51) International Patent Classification (IPC):
**A61N 5/10** *(2006.01)* **A61B 6/58** *(2024.01)*

(52) Cooperative Patent Classification (CPC):
**A61N 5/1075; A61B 6/583;** A61N 2005/1076

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **20.08.2024 US 202418809573**

(71) Applicant: **Siemens Healthineers International AG 6312 Steinhausen (CH)**

(72) Inventors:
• **Kieselmann, Jennifer**
 **5400 Baden (CH)**
• **Filiberti, Reto**
 **6340 Baar (CH)**
• **Thieme-Marti, Stefan**
 **5210 Windisch (CH)**

(74) Representative: **Mathisen & Macara LLP Charta House 30-38 Church Street Staines-upon-Thames TW18 4EP (GB)**

(54) **KINEMATIC CALIBRATION OF A TREATMENT COUCH FOR RADIATION THERAPY**

(57)   Example methods (400) and systems (190) for kinematic calibration of a treatment couch (131) for radiation therapy are described. In one example, a computer system (190) may generate and send (420) first instruction(s) to cause the treatment couch (131) to move towards each of multiple calibration poses. The treatment couch (131) may be associated with a kinematic model (530, 540). The computer system (190) may determine multiple measured poses that are associated with the respective multiple calibration poses. Next, the computer system (190) may determine (440) geometric correction data for updating the kinematic model (530, 540) based on the multiple measured poses. The computer system (190) may then generate and send second instruction(s) to update the kinematic model (530, 540) associated with the treatment couch (131) based on the geometric correction data.

| Degrees of Freedom (DoF)* | | |
|---|---|---|
| X | Lateral | 321 |
| Y | Longitudinal | 322 |
| Z | Vertical | 323 |
| $\theta_X$ | Pitch | 324 |
| $\theta_Y$ | Roll | 325 |
| $\theta_Z$ | Yaw | 326 |

* Examples: 3DoF, 4DoF, 6DoF, etc.

FIG. 3

EP 4 699 650 A1

**Description**

CROSS-REFERENCE TO RELATED APPLICATION

The present application (Attorney Case No. 09679) is related in subject matter to European Patent Application entitled "Numerical Calibration Approach for Treatment Couch Calibration" (Attorney Case No. 09678), with priority date 20 August 2024, and priority application number US18/809,563, which is incorporated herein by reference. The two applications are being filed on the same day.

BACKGROUND

Radiation therapy is a widely used cancer treatment modality that uses high-energy radiation to reduce or eliminate cancerous tumors. In practice, applied radiation does not inherently discriminate between a tumor and proximal healthy structures within a patient, such as organs, etc. Ideally, the objective is to deliver a lethal or curative radiation dose to the tumor, while maintaining an acceptable dose level in the proximal healthy structures. In practice, treatment couch calibration is performed to ensure precise and accurate delivery of radiation doses to the patient. Calibration helps to ensure that the treatment couch is positioned accurately during radiation therapy, such as relative to a linear accelerator (LINAC) that delivers radiation doses. Any inaccuracy associated with the positioning of the treatment couch, as well as that of the patient, may affect the accuracy and effectiveness of radiation treatment delivery.

BRIEF DESCRIPTION OF DRAWINGS

FIG. 1 is a schematic diagram illustrating an example radiation therapy system that includes a treatment delivery machine;

FIG. 2 is a schematic diagram illustrating a perspective view of the treatment delivery machine shown in the example in FIG. 1;

FIG. 3 is a schematic diagram illustrating an example detailed configuration of a couch positioning assembly shown in the example in FIGs. 1-2.

FIG. 4 is a flowchart of an example process for a computer system to perform kinematic calibration of a treatment couch for radiation therapy;

FIG. 5 is a schematic diagram illustrating example kinematic modelling for a treatment couch;

FIG. 6 is a flowchart of an example detailed process for a computer system to perform kinematic calibration of a treatment couch for radiation therapy;

FIG. 7 is a schematic diagram illustrating an example process for image acquisition of a calibration phantom and measured pose determination;

FIG. 8 illustrates a table of example geometric correction data for kinematic calibration; and

FIG. 9 is a schematic diagram illustrating an example radiation therapy system having a C-arm configuration.

SUMMARY

According to a first aspect of the invention, there is provided a method for a computer system to perform kinematic calibration of a treatment couch for radiation therapy, the method as defined by claim 1.
According to a second aspect of the invention, there is provided a computer system as defined by claim 6.
According to a third aspect of the invention, there is provided a radiation therapy system, as defined by claim 11.
Optional features are defined by the dependent claims.
According to examples of the present disclosure, treatment couch calibration may be performed in an improved manner using a kinematic calibration approach. In general, the term "kinematics" may refer generally to a branch of robotics or mathematics that focuses on describing the motion of objects. As used herein, the term "kinematic model" may refer generally to a representation (e.g., robotics or mathematical representation) that describes the motion of a moveable system, such as robot or treatment couch 131 for radiation therapy. Examples of the present disclosure should be contrasted against conventional approaches that necessitate manual adjustment(s) of a treatment couch during calibration, such as a technician mechanically adjusting axes of the treatment couch and/or treatment device, etc. Instead of necessitating mechanical adjustment(s), kinematic calibration may be implemented to update a kinematic model associated with the treatment couch, thereby calibrating the treatment couch programmatically. In practice, examples of the present disclosure may be performed to facilitate more accurate positioning of the treatment couch and patient during radiation therapy, which in turn leads to better treatment outcomes.

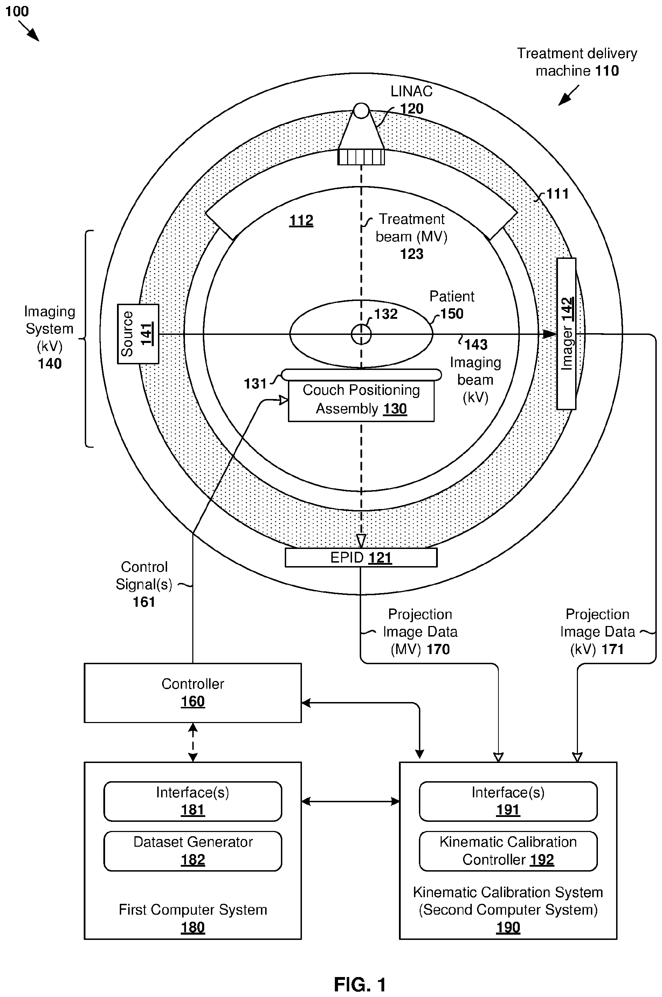

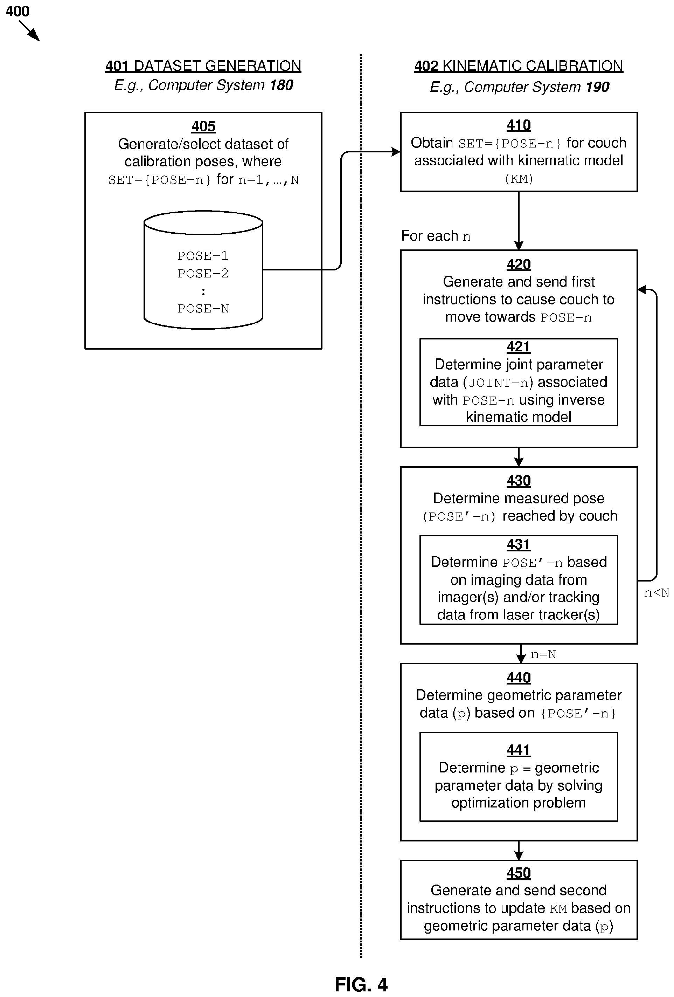

According to the first and second aspects, examples of the present disclosure provide method(s) and computer system(s) to perform kinematic calibration of a treatment couch for radiation therapy. In one example, a computer system (e.g., kinematic calibration system 190 in FIG. 1) may obtain a dataset of multiple calibration poses. Next, the computer system may generate and send first instruction(s) to cause the treatment couch to move towards each of multiple calibration poses in the dataset. See 410-420 in FIG. 4. The computer system may determine (e.g., measure) multiple measured poses that are associated with the respective multiple calibration poses. Based on the multiple measured poses, the computer system may determine geometric correction data for updating a kinematic model associated with the treatment couch. Based on the geometric correction data, the computer system may then generate and send second instruction(s) to update the kinematic model associated with the treatment couch. See 430-450 in FIG. 4.

According to the third aspect, examples of the present disclosure provide a radiation therapy system that includes (a) a treatment couch that is associated with a kinematic model and a computer system to perform kinematic calibration according to the first aspect or second aspect. Further aspects may include a non-transitory computer-readable storage medium that includes instruction(s) or program code which, in response to execution by a processor of a computer system, cause the processor to perform aspect(s) of the above method(s), as well as a computer system configured to implement aspect(s) of the above method.

DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the subject matter presented here. It will be readily understood that the aspects of the present disclosure, as generally described herein, and illustrated in the drawings, can be arranged, substituted, combined, and designed in a wide variety of different configurations, all of which are explicitly contemplated herein. Although the terms "first" and "second" are used to describe various elements, these elements should not be limited by these terms. These terms are used to distinguish one element from another. For example, a first element may be referred to as a second element, and vice versa. Independent of the grammatical term usage, individuals with male, female or other gender identities are included within the term.

Radiation therapy system

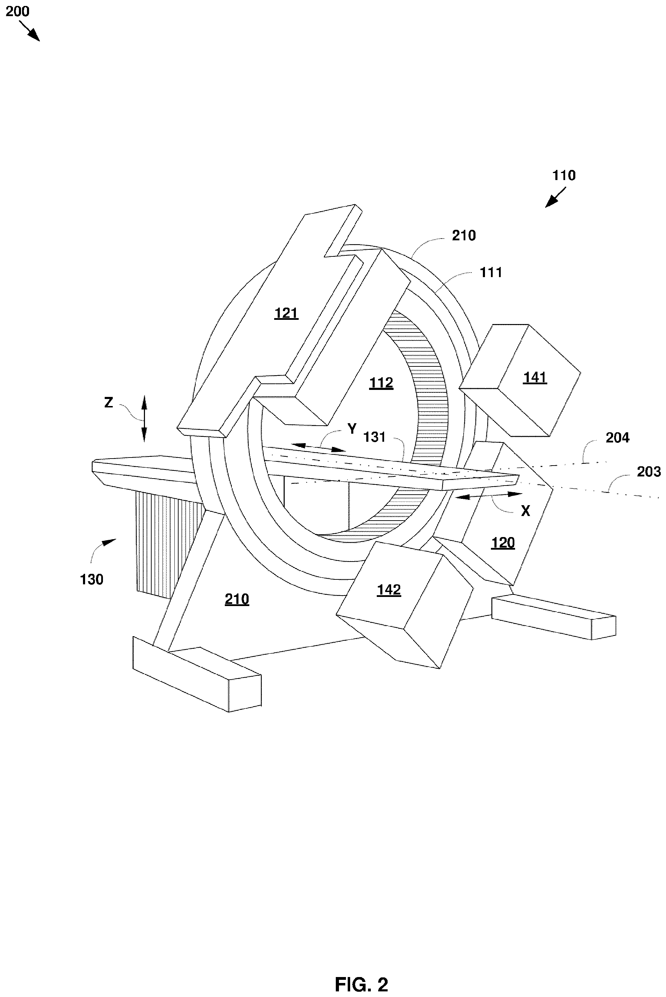

FIG. 1 is a schematic diagram illustrating example radiation therapy system 100 for delivery of radiation dose during a treatment phase of radiation therapy. FIG. 2 is a schematic diagram illustrating perspective view 200 of treatment delivery machine 110 in FIG. 1. It should be understood that, depending on the desired implementation, example system 100 and treatment delivery machine 110 may include additional and/or alternative components than that shown in FIGs. 1-2.

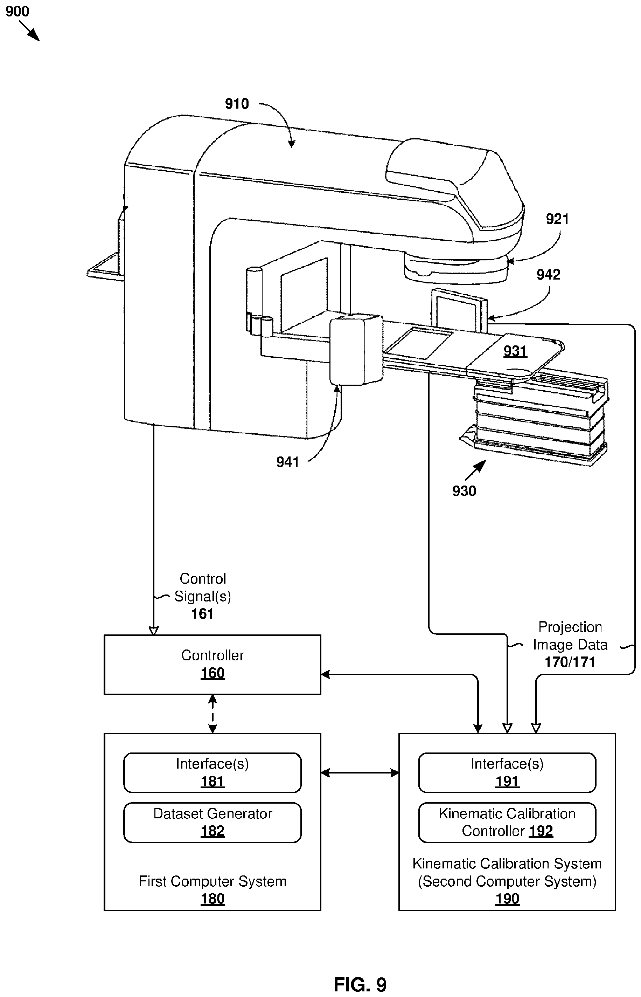

In the example in FIG. 1, radiation therapy system 100 may include (a) treatment delivery machine 110 to deliver treatment to patient 150, (b) control system or controller 160 to control operations of couch positioning assembly 130 to position patient 150, and (c) various computer systems 180-190 to facilitate treatment couch calibration according to examples of the present disclosure. Treatment delivery machine 110 may include gantry 111 that is rotatable about opening or bore 112 and treatment couch 131 for supporting patient 150 during radiation therapy. During treatment couch calibration, a calibration structure or device (known as a "phantom") may be positioned on treatment couch 131. As used herein, the term "treatment couch" may refer generally to a support structure (e.g., table) capable of supporting a patient or phantom. In practice, gantry 111 may have any suitable configuration, such as ring-based configuration (shown in FIGs. 1-2) or C-arm configuration (to be described using FIG. 9).

Treatment delivery machine 110 may include a radiation source in the form of linear accelerator (LINAC) 120 as well as an imager/detector in the form of mega-electron volts (MV) electronic portal imaging device (EPID) 121. LINAC 120 may be configured to generate and direct treatment beam 123 towards isocenter 132 as gantry 111 is rotated through a treatment arc during VMAT. In practice, isocenter 132 may refer generally to a point at which beam trajectories associated with different gantry positions converge or intersect. Treatment beam 123 may be within a high-energy range, such as 1 MV or greater to deliver radiation doses to any suitable target structure(s) associated with patient 150, such as tumor, etc. Couch position assembly 130 may be configured to position treatment couch 131 and patient 150 during radiation therapy. Couch position assembly 130 may position treatment couch 131 when the system is offline, for example when radiation therapy is not being performed. For example, treatment couch 131 may be positioned such that a target structure (e.g., tumor) associated with patient 150 is at or near isocenter 132 about which LINAC 120, EPID 121, X-ray source 141, and X-ray imager 142 are rotated.

Treatment delivery machine 110 may further include imaging system 140 to facilitate kV imaging. Any suitable image modality or modalities may be used, such as cone beam computed tomography (CBCT), etc. Imaging system 140 may include at least one pair of kV imaging source 141 and kV imager 142. Compared to LINAC 120, kV imaging source 141 may be capable of producing imaging or diagnostic energy in the range of kV. To generate kV projection image data, kV imaging source 141 may be configured to emit and direct kV imaging beam 143 towards imager 142 to image patient 150. Although described with reference to MV LINAC 120 and MV treatment beam 123, it should be understood that any additional or alternative treatment delivery technique(s) may be used. For example, a proton treatment machine that includes a kV imaging system may be used instead.

Referring also to FIG. 2, various elements of treatment delivery machine 110 (e.g., LINAC 120, EPID 121, kV source 141, and kV imager 142) are shown to be partially rotated about treatment couch 131 at a specific point in time. Here, treatment delivery machine 110 may further include a drive stand 210 that is mechanically coupled to couch positioning assembly 130. In practice, drive stand 210 may be a fixed support structure for components of treatment delivery machine 110, including gantry 111, a drive system (not shown) for rotatably moving gantry 111, cooling systems (not shown) of treatment delivery machine 110, etc. Drive stand 210 may rest on and/or be fixed to a support surface, such as a floor of a treatment facility. In the example in FIG. 2, couch positioning assembly 130 may be mechanically coupled to drive stand 210 and configured to adjustably position treatment couch 131 relative to bore 112 and LINAC 120. Treatment couch 131 is shown to be longitudinally extending from couch positioning assembly 130 into a bore 112 of treatment delivery machine 110.

Couch positioning assembly

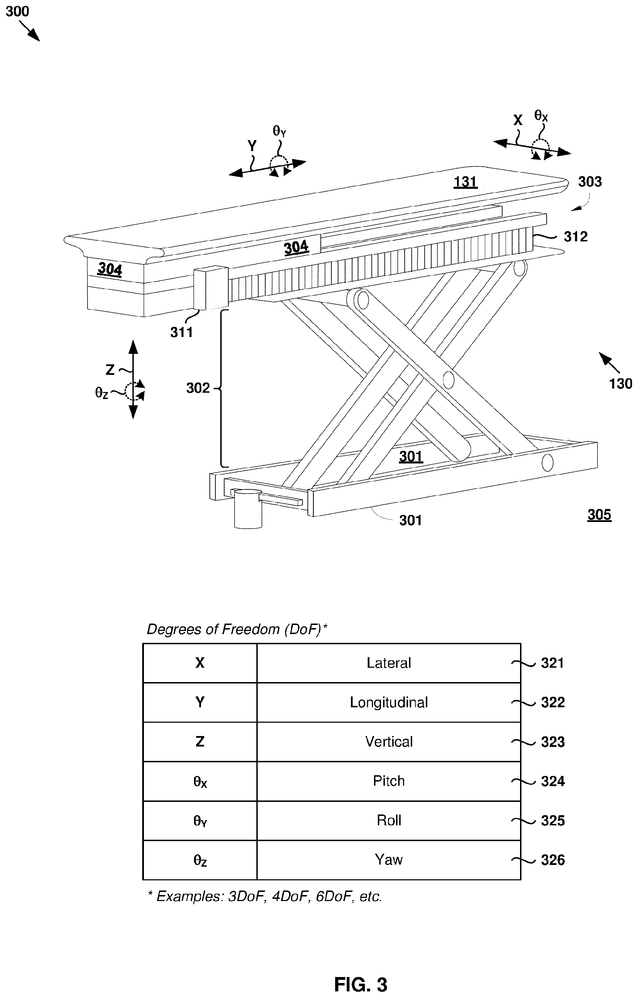

FIG. 3 is a schematic diagram illustrating example detailed view 300 of couch positioning assembly 130 in the example in FIGs. 1-2. Here, couch positioning assembly 130 is depicted without external panels (shown in FIG. 2) for clarity. In the example in FIG. 3, couch positioning assembly 130 may include lift base 301 on which scissor lift mechanism 302 and longitudinal frame 303 are mounted. In this example, lift base 301 may be configured to translate treatment couch 131 in lateral directions (X). Coupled to longitudinal frame 303 is a longitudinal carriage 304 that may be translated along longitudinal directions (Y) via longitudinal motor 311. Treatment couch 131 may be mounted on, and longitudinally moved by, longitudinal carriage 304. Scissor lift mechanism 302 may be configured to translate treatment couch 131 in vertical directions (Z) using any technically feasible actuation system.

Depending on the desired implementation, couch positioning assembly 130 and treatment couch 131 may support movements in any suitable number of degrees of freedom (DoF), such as three (3DoF), four (4DoF), six (6DoF), etc. In the case of 3DoF, couch positioning assembly 130 may be configured to translate treatment couch 131 along (X, Y, Z) directions, where X = lateral directions (see 321), Y = longitudinal directions (see 322), and Z = vertical directions (see 323). The three primary axes are also shown in FIG. 2. Movement of treatment couch 131 along lateral directions X corresponds to horizontal movement towards one side or the other side (e.g., left or right) of bore 112. Movement along longitudinal directions Y corresponds to horizontal movement into or out (e.g., forward or backward) of bore 112. Movement along vertical directions Z corresponds to vertical movement towards or away from (e.g., down or up) treatment room floor 305.

In the case of 4DoF, movements about one rotational axis are supported in addition to three translational axes. In this case, couch positioning assembly 130 may be configured to position treatment couch 131 along (X, Y, Z, θ) directions, where θ = pitch associated with rotation about a lateral (X) axis, θ = rotation (e.g. roll) about the Y-axis, or θ = yaw associated with rotation about the Z-axis. In practice, θ = yaw is often used for radiation treatments. Compared to 3DoF, a 4DoF couch provides more flexibility in adjusting patient position, such as for noncoplanar treatments. See corresponding 321-323 and 324/325/326 in FIG. 3.

In the case of 6DoF, movements about three translational axes and three rotational axes are supported. In this case, couch positioning assembly 130 may be configured to position treatment couch 131 along $(X, Y, Z, \theta_X, \theta_Y, \theta_Z)$ directions, where $\theta_X$ = pitch associated with rotation about the lateral X-axis, $\theta_Y$ = roll associated with rotation about the longitudinal Y-axis, and $\theta_Z$ = yaw associated with rotation about the vertical Z-axis. Compared to a 3DoF or 4DoF couch, a 6DoF couch may combine pitch, roll and yaw rotational motions with translational motions to achieve high-precision patient positioning, such as in the order of submillimeter and sub-degree precision. See corresponding 321-326 in FIG. 3.

In practice, couch positioning assembly 130 may include any suitable motors and joints (not all shown in FIG. 3 for simplicity). To support movement in lateral directions X, couch positioning assembly 130 may include a lateral movement motor (e.g., linear motor) for selectively moving treatment couch 131 in lateral directions *X*. To support movement in longitudinal directions *Y*, couch positioning assembly 130 may include a longitudinal movement motor (e.g., linear motor) that is coupled with longitudinal frame 303 for selectively moving treatment couch 131 in longitudinal directions *Y*. To support movement in vertical directions Z, couch positioning assembly 130 may include scissor lift mechanism 302 for moving treatment couch 131 using any suitable actuation system, such as may be an electric motor, a hydraulic actuator, a stepper motor, etc. To support rotational motions in $(\theta_X, \theta_Y, \theta_Z)$ directions, couch positioning assembly 130 may include any suitable rotational motors to enable pitch, roll and yaw adjustments.

Couch positioning assembly 130 may be controlled using controller 160 in FIG. 1 using any suitable commands and control signals (see 161 in FIG. 1). For quality assurance (QA) purposes or in general, treatment couch calibration may be performed prior to treatment delivery, for example such that the actual movement of treatment couch 131 better matches with or follows the commands from controller 160. For example, when 6DoF couch 131 is instructed to move towards a particular target position (known as "pose"), the reached position might differ from the target position. In an example, a "pose" may correspond to particular values of the lateral, longitudinal and/or vertical positions of the couch 131, and particular values of the pitch, roll and/or yaw of the couch 131. The discrepancy may be due to various imperfections associated with couch 131, for example due to manufacturing tolerances. This is undesirable because, in some cases, even minor discrepancies may lead to inaccurate radiation delivery to a target structure (e.g., tumor), missing part of the target, and inadvertently harming healthy tissues of patient 150.

Kinematic calibration

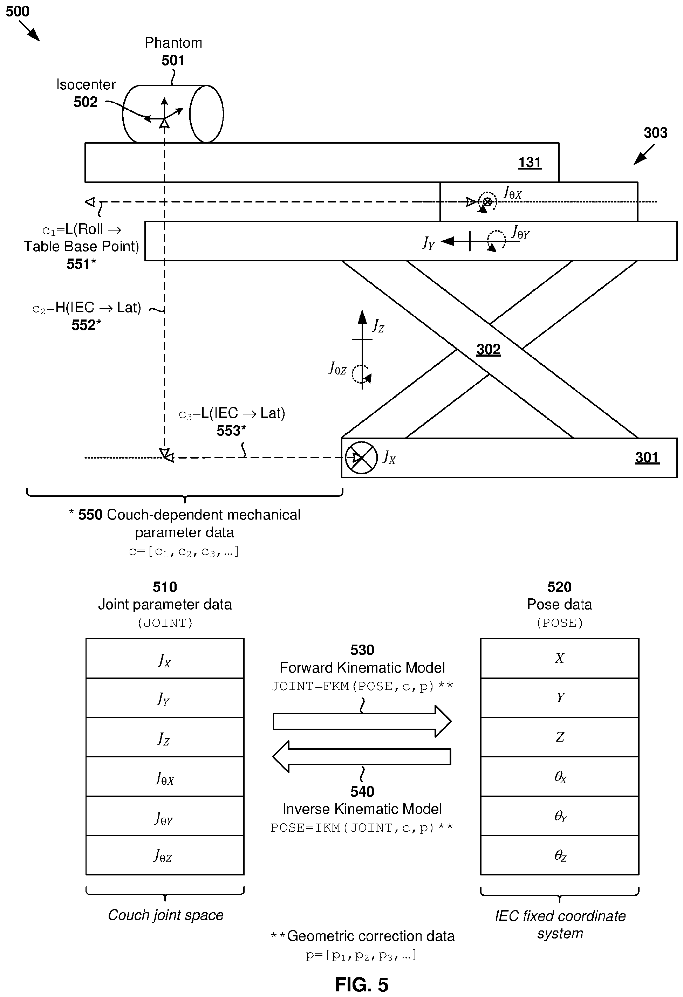

According to examples of the present disclosure, treatment couch calibration may be performed in an improved manner using a kinematic calibration approach. In particular, examples of the present disclosure may involve updating a kinematic model (to be discussed further using FIG. 5) associated with treatment couch 131. Examples of the present disclosure should be contrasted against conventional calibration approaches that necessitate manual adjustment(s) to treatment couch 131 to correct its end position. Instead of performing manual adjustment(s), the kinematic model may be updated programmatically to correct or alleviate couch imperfection(s) due to machining, assembling, as well as installation or mounting tolerances.

Some examples will be described using FIG. 4, which is a flowchart of example process 400 for first computer system 180 to perform calibration dataset generation and second computer system 190 (refer to FIG. 1) to perform treatment couch calibration. Example process 400 may include one or more operations, functions, or actions illustrated by one or more blocks, such as 405 to 450. Depending on the desired implementation, various blocks may be combined into fewer blocks, divided into additional blocks, and/or eliminated. In the example in FIG. 4, treatment couch calibration may include two phases: (a) calibration dataset generation 401 to generate/select a dataset of calibration poses (see block 405) and (b) on-site kinematic calibration 402 based on the generated dataset (see blocks 410-450).

Examples of the present disclosure may be implemented using any suitable computer system(s). As used herein, the term "computer system" may refer generally to one or more physical machines (bare metal machines), or virtual machines (VMs) deployed in a cloud-based environment. Referring to FIG. 1 again, computer systems 180-190 may be communicatively coupled with treatment delivery machine 110 to facilitate couch calibration. Note that computer systems 180-190 may be implemented using one or more physical/virtual machines. Further, computer system 180/190 may be located in the same, or a different, physical location as treatment delivery machine 110.

Using the example in FIG. 1, first computer system 180 may be configured to perform calibration dataset generation 401 according to blocks 405 in FIG. 4. First computer system 180 may include any suitable hardware and/or software components, such as interface 181 to interact with controller 160 and/or second computer system 190, and dataset generator 182 to perform block 405. Second computer system 190 (i.e., kinematic calibration system) may be configured to perform couch calibration 402 according to blocks 410-450 in FIG. 4. Second computer system 190 may include any suitable hardware and/or software components, such as interface 191 to interact with controller 160 and/or first computer system 180, kinematic calibration controller 192 to perform blocks 410-450. Phases 401-402 will be described in turn below.

(a) Dataset generation (see 401)

At 405 in FIG. 4, first computer system 180 may perform dataset generation to generate a dataset of multiple (*N*) calibration poses for use during kinematic calibration. The dataset may be denoted as *SET* = {*POSE- n*} , where *POSE- n* represents a particular calibration pose and *n* = 1, ..., *N*. As used herein, the term "pose" may refer generally to a position associated with a treatment couch. The term "calibration pose" or "calibration measurement position" may refer generally to a position that a treatment couch is instructed to reach during couch calibration. As will be explained using FIG. 5, a calibration pose may be expressed using coordinates in a machine coordinate space, such as the International Electrotechnical Commission (IEC) fixed coordinate system, etc.

Depending on the desired implementation, block 405 may include performing a numerical calibration approach to select the dataset from multiple (*M*) candidate datasets (e.g., *SET*-1, ..., *SET-M*). Example implementations of the numerical calibration approach may be found in related European Patent Application entitled "Numerical Calibration Approach for Treatment Couch Calibration" (Attorney Case No, 09678) with priority date 20 August 2024, and priority application number US18/809,563. For example, the dataset may be selected based on metric data according to any desired selection criterion or criteria relating to calibration accuracy and/or calibration time. Depending on the desired implementation, calibration poses in the dataset may be generated randomly.

(b) Kinematic calibration based on dataset (see 402)

At 410 in FIG. 4, second computer system 190 (kinematic calibration system) may obtain a dataset denoted as *SET* = {*POSE- n*} for *n* = 1, ..., *N* from first computer system 180 or any suitable datastore. At 420, based on *SET* = {*POSE-n*}, computer system 190 may generate and send first instruction(s) to cause treatment couch 131 to move towards each of the multiple (*N*) calibration poses in *SET*. Depending on the desired implementation, the first instruction(s) may be generated and sent to controller 160 that is communicatively coupled with couch positioning assembly 130. In response to receiving the first instruction(s), controller 160 may instruct couch positioning assembly 130 to move treatment couch 131 towards a particular *POSE- n* (see 161 in FIG. 1). In an alternative embodiment, the first instruction(s) may be generated and sent to couch positioning assembly 130 directly.

In practice, treatment couch 131 may move towards a particular calibration pose by moving its motors and/or joints that are part of couch positioning assembly 130 in the example in FIG. 3. As will be described further using FIGs. 5-6, controller 160 or computer system 190 may cause treatment couch 131 to move based on joint parameter data (denoted as *JOINT-n*) associated with a particular calibration pose (*POSE- n*). In this case, controller 160 or computer system 190 may determine *JOINT-n* based on a kinematic model associated with treatment couch 131. The kinematic model may include (a) an inverse kinematic model and (b) a forward kinematic model. The inverse kinematic model is for mapping or transforming a particular pose (e.g., *POSE- n*) to joint parameter data (e.g., *JOINT-n*). The forward kinematic model is for mapping *JOINT-n* to *POSE- n*. See also 421 in FIG. 4 and 530-540 in FIG. 5. In an example, the forward kinematic model may determine pose from joint parameter data. In an example, the inverse kinematic model may determine joint parameter data from pose.

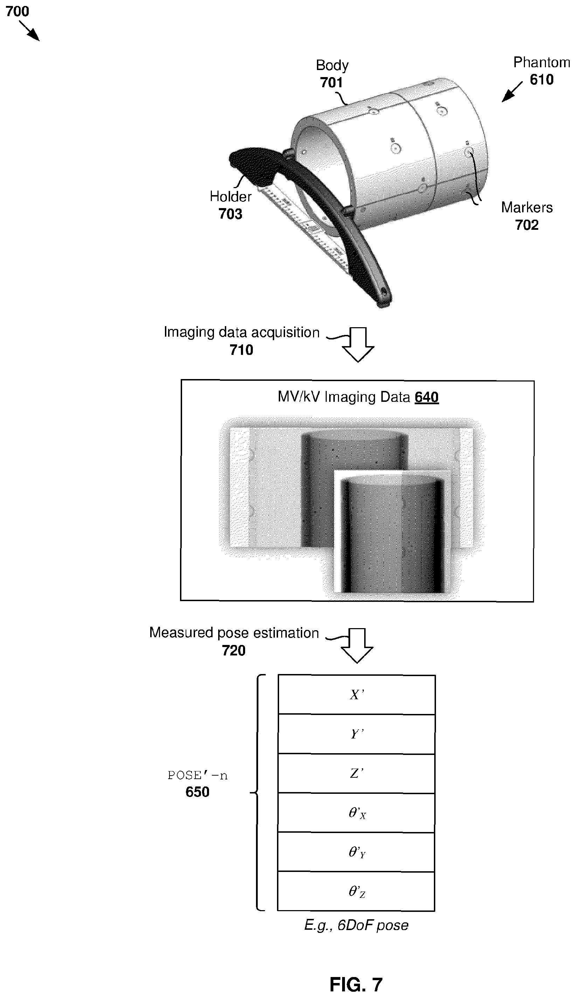

At 430 in FIG. 4, second computer system 190 may determine multiple (*N*) measured poses that are reached by treatment couch 131. The measured poses (denoted as {*POSE'-n*}) are associated with the respective multiple (*N*) calibration poses. As used herein, the term "reached pose" or "measured pose" may refer generally to a position that is actually achieved by the treatment couch, as measured using any suitable measurement approach. For example, the measured poses may be determined based on MV imaging data 170 from EPID 121 and/or kV imaging data 171 from kV imager(s) 142. Alternatively or additionally, the measured poses may be determined based on tracking/positioning data from laser tracker(s). See also 431 in FIG. 4. Example measured pose determination will be explained using FIG. 7.

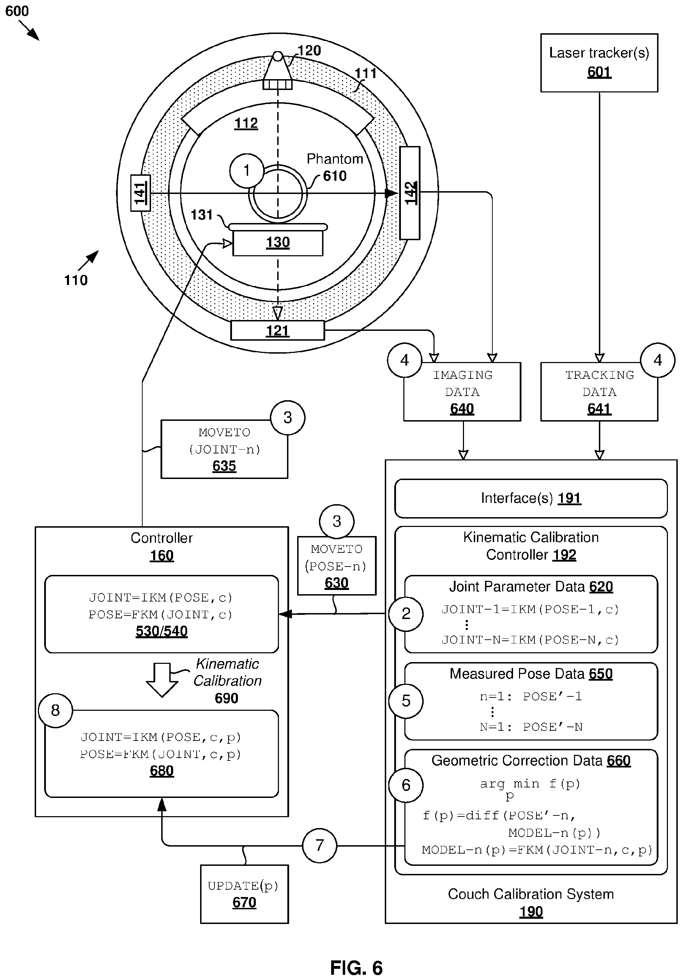

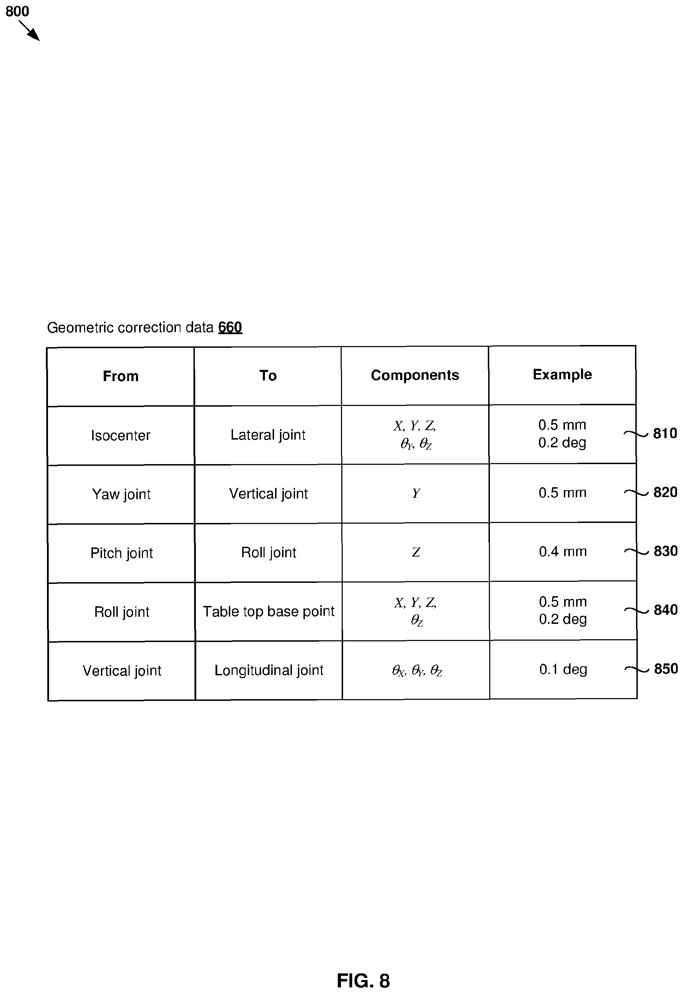

At 440 in FIG. 4, based on the measured poses, second computer system 190 may determine geometric correction data (*p*) for updating the kinematic model associated with treatment couch 131. As used herein, the term "geometric correction data" or "geometric parameter data" associated with a couch may refer generally to one or more linear and/or rotational adjustment(s) that may be made to a treatment couch. Block 440 may involve solving an optimization problem to determine the geometric correction data (*p*) that minimizes deviation data associated with (a) the measured poses estimated at block 430 and (b) modelled poses parameterized by *p* (to be explained further using FIG. 6). See also 441 in FIG. 4. Example geometric correction data (*p*) will be explained using FIG. 8.

At 450 in FIG. 4, computer system 190 may generate and send second instruction(s) to cause treatment couch 131 to update its kinematic model based on the geometric correction data (*p*). Depending on the desired implementation, the second instruction(s) may be generated and sent to controller 160. In response to receiving the second instructions, controller 160 may generate and send control signal(s) 161 to couch positioning assembly 130 to update its kinematic model. In an alternative embodiment, the second instruction(s) may be generated and sent to couch positioning assembly 130 directly. Various examples will be discussed below.

Kinematic Modelling

According to examples of the present disclosure, treatment couch calibration may be performed based on a kinematic model. In general, the term "kinematics" may refer generally to a branch of robotics or mathematics that focuses on describing the motion of objects. As used herein, the term "kinematic model" may refer generally to a representation (e.g., robotics or mathematical representation) that describes the motion of a moveable system, such as robot or treatment couch 131 for radiation therapy. In the following, various examples will be explained using treatment couch 131 supporting 6DoF (see 321-326 in FIG. 3). In practice, examples of the present disclosure may be implemented for treatment couch 131 supporting any degrees of freedom, such as 3DoF, 4DoF, etc.

An example will be explained using FIG. 5, which is a schematic diagram illustrating example kinematic modeling 500 for treatment couch calibration. Here, a side view of treatment couch 131 and couch positioning assembly 130 is shown. One goal of treatment couch calibration may include determining a kinematic model that describes, as close as possible to reality, a relationship or conversion between (a) couch motor and/or joint settings associated with treatment couch 131 and (b) its so-called end-effector pose. In an example, conversion from couch motor and/or joint settings to an end-effector pose may be performed by a forward kinematic model. In an example, conversion from an end-effector pose to couch motor and/or joint settings may be performed by an inverse kinematic model. That means, for a desired end-effector pose, each couch joint may move in such a way that the pose is reached within certain tolerance values.

(a) Couch joint space

At 510 in FIG. 5, couch motor and/or joint parameter data (denoted as $JOINT$) may be expressed in a couch joint space. For brevity, the term "couch joint parameter data" will be used to represent couch motor and/or joint settings. One example is $JOINT = (J_X, J_Y, J_Z, J_{qX}, J_{qY}, J_{qZ})$, where $(J_X, J_Y, J_Z)$ may represent motor or joint parameter data associated with translational motion in the lateral direction $X$, longitudinal direction $Y$ and vertical direction $Z$ in the couch joint space, respectively. Further, $(J_{qX}, J_{qY}, J_{qZ})$ may represent motor or joint parameter data associated with rotational motion about a lateral axis, a longitudinal axis and a vertical axis in the couch joint space, respectively. As described using FIG. 3, couch positioning assembly 130 may include lift base 301, scissor lift mechanism 302 and longitudinal frame 303 to move treatment couch 131 in translational and rotational directions.

(b) IEC fixed coordinate system

At 520 in FIG. 5, an end-effector pose (denoted as $POSE$) may be expressed in the IEC fixed coordinate system. One example is $POSE = (X, Y, Z, \theta_X, \theta_Y, \theta_Z)$, where $(X, Y, Z)$ may represent translational movements in respective lateral axis $X$, longitudinal direction $Y$ and vertical direction $Z$ in the IEC fixed coordinate system. Further, $(\theta_X, \theta_Y, \theta_Z)$ may be angles representing rotational movements about respective lateral X-axis, longitudinal $Y$-axis and vertical Z-axis in the IEC fixed coordinate system, respectively. In practice, one way to model the end-effector pose is by way of the origin and orientation of a calibration phantom (see 501 in FIG. 5) that is placed on treatment couch 131.

(c) Forward and inverse kinematic models

A kinematic model describes the conversion between (a) couch joint parameter data ($JOINT$) in the couch joint space and (b) an end-effector pose ($POSE$) in the IEC fixed coordinate system in both directions. At 530 in FIG. 5, a forward kinematic model (FKM) for mapping or converting $JOINT$ in the couch joint space to $POSE$ in the IEC fixed coordinate system may be expressed as follows using $c$ = mechanical parameter data and $p$ = geometric correction data (to be described below) associated with treatment couch 131:

$$\text{FKM}(JOINT, c, p) = \text{FKM}(J_X, J_Y, J_Z, J_{qX}, J_{qY}, J_{qZ}, c, p) = (X, Y, Z, \theta_X, \theta_Y, \theta_Z) = POSE.$$

At 540 in FIG. 5, an inverse kinematic model (IKM) for mapping or converting (a) an end-effector pose ($POSE$) in the IEC fixed coordinate system to (b) couch joint parameter data ($JOINT$) in the couch joint space may be expressed as follows using $c$ = mechanical parameter data and $p$ = geometric correction data (to be described below) associated with treatment couch 131:

$$\text{IKM}(POSE, c, p) = \text{IKM}(X, Y, Z, \theta_X, \theta_Y, \theta_Z, c, p) = (J_X, J_Y, J_Z, J_{qX}, J_{qY}, J_{qZ}) = JOINT.$$

(e) Mechanical parameter data

At 550 in FIG. 5, mechanical parameter data ($c$) for the FKM and IKM may include any parameter(s) associated with treatment couch 131, such as nominal geometric relations between various joints. At 551, a first example (c1) is H($IEC{\rightarrow}Lat$), which represents the height (H) or vertical distance from an isocenter in the IEC fixed coordinate system (see 502 in FIG. 5) to a lateral joint. At 552, a second example (c2) is L($IEC{\rightarrow}Lat$), which represents a longitudinal distance from the isocenter ($IEC$) to a lateral joint ($Lat$). At 553, a third example (c3) is L($Roll{\rightarrow}Base\ Point$), which represents a longitudinal distance between a roll joint ($Roll$) and a tabletop base point ($Base\ Point$) of treatment couch 131. Other examples may include H($Lng{\rightarrow}Pitch$) = vertical distance from a longitudinal joint ($Lng$) to a pitch joint ($Pitch$), L($Vrt{\rightarrow}Lng$) = longitudinal distance from a vertical joint ($Vrt$) to a longitudinal joint ($Lng$), etc.

In practice, the mechanical parameter data is different for each individual couch. While the mechanical parameter data is expected to stay within defined tolerances of the manufacturing process of treatment couch 131, some deviations from nominal values are expected. These deviations may be caused by various imperfections, such as offsets from the nominal lengths of parts of treatment couch 131, misaligned axes (e.g., longitudinal motion direction is not orthogonal to the vertical motion direction), a misaligned object on treatment couch 131 (i.e., the end-effector). To account for these deviations, geometric corrections (denoted as $p$) may be introduced to the kinematic model. These geometric corrections, which are specific for individual couches, are unknown parameters in the model. As will be explained further using FIGs. 6-8, the process of kinematic calibration may include finding these geometric corrections and updating the kinematic model based on these geometric corrections accordingly.

In practice, the forward and inverse kinematic models may include any suitable transformation functions or matrices for transform *JOINT* into *POSE,* and vice versa. For example, given a moveable system (e.g., robot or couch) with three so-called prismatic joints (e.g. moving in lateral, longitudinal and vertical directions), the model may be described as follows. Let $d_k$ = movable joint variable of a particular joint $k$ and $p_k$ = geometric correction(s), the forward kinematic can be described as: $f(d_1, d_2, d_3, p) = A_1(d_1, p_1) * A_2(d_2, p_2) * A_3(d_3, p_3)$, where $A_i$ is the transformation matrix of joint $k$.

For instance, using the Denavit-Hartenberg representation in the field of robotics, each joint may be described by one free parameter (e.g., $d_k$) and two fixed parameters, such as link length offset $a_k$ (i.e. the distance between two consecutive joints in the chain), and link twist offset $\alpha_k$ (i.e. the angle between the common normal of two consecutive joints). The parameters $p_k$ may be, for instance, offsets to the fixed parameters $a_k$ and $\alpha_k$. For the inverse kinematics and a requested end-effector pose $(X_r, Y_r, Z_r)$, a set of equations may be defined based on the forward kinematics as $f(d_1, d_2, d_3, p) = (X_r, Y_r, Z_r)$. This equation system may be solved numerically, as a closed-form analytical solution is generally not available when geometric corrections are involved. These examples discussed with reference to the lateral, longitudinal and vertical directions are also applicable to rotational directions.

<u>Detailed example</u>

FIG. 6 is a flowchart of example detailed process 600 for computer system 190 to perform kinematic calibration of treatment couch 131 for radiation therapy. Example process 600 may include one or more operations, functions, or actions illustrated by one or more blocks, such as 610 to 680. Depending on the desired implementation, various blocks may be combined into fewer blocks, divided into additional blocks, and/or eliminated. The example in FIG. 6 will be explained using FIG. 7, which is a schematic diagram illustrating example process 700 for image acquisition of a calibration phantom and measured pose determination.

(a) Calibration phantom

At 610 in FIG. 6, a calibration phantom may be manually placed on treatment couch 131, such as by a technician or clinician who is involved in couch calibration. In practice, any phantom of suitable shape and size may be used. For example in FIG. 7, phantom 610 may have tubular or cylindrical body 701 that is attached with multiple X-ray-visible features in the form of markers 702. Markers 702 may be embedded within a wall or secured to a surface (e.g., exterior) of cylindrical body 701. Markers 702 may include multiple (e.g., 16) bearing balls that are made of steel and arranged in an asymmetric pattern. Alternatively or additionally, marker(s) may be embedded in a couch top. Such a couch top with marker(s) may be fixedly installed. A particular marker may be installed on one insert of a couch-top system with multiple inserts.

Phantom 610 may include holder 703 to secure cylindrical body 701 to treatment couch 131. Depending on the desired implementation, one or multiple reflectors (not shown) may be used for laser-tracker measurements, such as by placing the reflectors onto treatment couch 131 directly. In practice, any suitable alternative to the multiple reflectors may be used for tracking purposes. Phantom 610 may be aligned at a virtual isocenter with the laser trackers and moved to a treatment isocenter. This is used as a reference position. With knowledge of the relative positioning of markers 702 on phantom 610 and/or reflectors attached to holder 703, poses of phantom 610 and therefore treatment couch 131 may be measured based on MV/kV imaging data and/or tracking data (to be explained below).

(b) First instruction(s)

At 620 in FIG. 6, based on *SET* = {*POSE-n*}, computer system 190 may determine joint parameter data (*JOINT-n*) associated with various motors and/or joints of treatment couch 131 using an ideal inverse kinematic model. In particular, for each *POSE-n*, *JOINT-n = IKM(POSE-n, c)* may be determined based on any suitable *c* = mechanical parameter data (see 550 in FIG. 5) associated with treatment couch 131 and no geometric correction (i.e., $p = 0$ for an uncalibrated couch).

At 630 in FIG. 6, computer system 190 may generate and send first instruction(s) to controller 160 to cause treatment couch 131 to move towards each *POSE-n* from *SET* = {*POSE-n*}, where $n = 1, ..., N$. Here, the term "instruction" may refer generally to a command or control signal that is readable or executable by controller 160. In one example, the first instruction(s) may include command(s) identifying *POSE-n,* in which case controller 160 may map *POSE-n* to *JOINT-n* using inverse kinematic model 530 accordingly. In this case, block 620 may be skipped. Alternatively, the first instruction(s) may include command(s) identifying *JOINT-n* calculated at block 620, in which case controller 160 does not have to calculate the joint parameter data. In both cases, controller 160 may cause couch positioning assembly 130 and therefore treatment couch 131 to move towards *POSE-n* based on *JOINT-n.* See 635 in FIG. 6.

(c) Measured pose determination

At 640-650 in FIG. 6, based on imaging data 640 and/or tracking data 641, computer system 190 may determine a measured pose (*POSE'-n*) that is reached by treatment couch 131 in response to instruction(s) to move to an associated calibration pose (*POSE-n*). Tracking data 641 may be acquired using laser tracker(s) 601 installed in the treatment room. Imaging data 640 may include first projection image data (MV) from EPID 121 and/or second projection image data (kV) from imager 142.

In the example in FIG. 7, image acquisition 710 may be performed during calibration to generate imaging data 640 that includes MV/kV image pairs or MV/MV image pairs. Using drum phantom 610, a 6DoF measured pose *POSE'-n* = (*X', Y', Z', $\theta'_X$, $\theta'_Y$, $\theta'_Z$*) in the IEC fixed coordinate system may be estimated, such as using a fitting algorithm, etc. Due to imperfections associated with treatment couch 131, there is usually a discrepancy between the target calibration pose (*POSE-n*) and the actual reached pose (*POSE'-n*).

Geometric correction data

At 660 in FIG. 6, based on measured poses {*POSE'-n*} associated with respective calibration poses {*POSE-n*}, computer system 190 may determine geometric correction data (*p*) for updating kinematic models 530-540. Block 660 may involve solving an optimization problem to determine geometric correction data (p) to minimize or reduce deviation data between (a) the measured poses (*POSE'-n*) and (b) modelled poses (denoted as *MODEL-n*) that are parameterized by *p*. The modelled poses may represent expected calibration positions. The optimization problem may be formulated as follows:

$$\arg\min_{p} f(p), \text{ where } f(p) = diff\big(POSE'\text{-}n, MODEL\text{-}n(p)\big).$$

In the above formulation, a particular modelled pose parameterized by *p* may be calculated based on forward kinematic model 530 as follows: *MODEL-n(p)* = *FKM(JOINT-n, c, p)*. Given a 3DoF measured *POSE'-n* = (*X', Y', Z'*) and modelled pose *MODEL-n* = (*X(p), Y(p), Z(p)*), the deviation data may be estimated using the following:

$$f(p) = \sqrt{\big(X' - X(p)\big)^2 + \big(Y' - Y(p)\big)^2 + \big(Z' - Z(p)\big)^2}.$$

The above *f(p)* may be extended to include rotational DoF(s) for a particular 6DoF measured *POSE'-n* = (*X', Y', Z', $\theta'_X$, $\theta'_Y$, $\theta'_Z$*) and modelled pose *MODEL-n* = (*X(p), Y(p), Z(p), $\theta_X(p)$, $\theta_Y(p)$, $\theta_Z(p)$*). Any suitable *f(p)* may be used. For example, to estimate the deviation data, angular deviations may be weighed against linear deviations, angular distances calculated using quaternions, etc.

In practice, any suitable formula(s) may be used to estimate the deviation data. Depending on the desired implementation, the optimization problem may be solved using any suitable approach, such as Monte Carlo simulation, simulated annealing approach, conjugate gradient approach, linear programming, dynamic programming, machine learning approach, etc. Various optimization approaches may be found in Numerical Recipes: The Art of Scientific Computing (by W. H. Press, S. H. Teukolsky, W. T. Vetterling and B. P. Flannery), which is incorporated herein by reference.

Depending on the desired implementation, a particular geometric correction or geometric parameter (*$p_k$*) may be associated with a position of a component of couch positioning assembly 130, a distance between two components (e.g., joint-to-joint distance), an orientation of a component, an axis of rotation, a center of rotation, a distance between an isocenter to a particular component, etc. FIG. 8 illustrates table 810 of example geometric correction data. At 810, a first example geometric correction (*$p_1$*) may be an offset to a linear or angular distance (e.g., 0.5 mm or 0.2 degrees) between isocenter 502 and a lateral joint of treatment couch 131. At 820, a second example (*$p_2$*) may be an offset to a distance (e.g., 0.5 mm) between a yaw joint and a vertical joint. At 830, a third example (*$p_3$*) may be an offset to a distance (e.g., 0.5 mm) between a pitch joint and a roll joint. At 840, a fourth example (*$p_4$*) may be an offset to a linear or angular distance (e.g., 0.5 mm or 0.2 degree) between a roll joint and a table top base point. At 850, a fifth example (*$p_5$*) may be an offset to an angular distance (e.g., 0.2 degree) between a vertical joint and a longitudinal joint. In practice, the geometric correction data (p) that is determined at block 660 may include any suitable number (K) parameters ($p_1, \dots , p_K$).

Updated kinematic model

Referring to FIG. 6 again, at 670, computer system 190 may generate and send second instruction(s) to controller 160 to update a kinematic model associated with treatment couch 131 based on geometric correction data (p), thereby calibrating treatment couch 131 kinematically. The second instruction(s) may be in any suitable form that is interpretable by controller 160, such as command(s) in programming or machine language, application programming interface (API) call(s), etc. The kinematic model to be updated may include inverse and forward kinematic models 530-540.

At 680 in FIG. 6, updated inverse and forward kinematic models may be expressed as follows based on $c$ = mechanical parameter data and $p$ = geometric correction data:

$$JOINT = IKM(POSE, c, p) \text{ and } POSE = FKM(JOINT, c, p).$$

Once updated, controller 160 may be capable of mapping between (a) a particular pose (POSE) and (b) joint parameter data (JOINT) using updated kinematic models 680 instead of pre-calibrated kinematic models 530-540. The update may be performed to improve the positioning of treatment couch 131 using couch positioning assembly 130 during radiation treatment. Using examples of the present disclosure, kinematic calibration may be performed to replace or reduce the need for mechanical adjustment(s) of treatment couch 131 based on geometric correction data ($p$). See also 690 in FIG. 6.

Depending on the desired implementation, treatment couch 131 is usually calibrated over its full travel range, whether indirectly (e.g., calibrating only one part of the range but verifying that the full range is calibrated) or directly (e.g., calibrating explicitly the full range). To ease calibration, it is envisioned that the kinematic model may be calibrated by placing phantom 610 on one position of couch 131. To determine whether this hypothesis holds, the evaluation of the calibrated couch may be performed on multiple positions of phantom 610 relative to couch 131. Alternatively, phantom 610 may be placed on multiple positions on treatment couch 131, in which case kinematic calibration is performed for each phantom position. For example, one may move phantom 610 when the full travel range of treatment couch 131 cannot be covered using a particular imaging system.

Example C-arm configuration

Although discussed using radiation therapy system 100 having a ring-based configuration, any alternative configuration may be used. For example, FIG. 9 is a schematic diagram illustrating example radiation therapy system 900 having a C-arm configuration. In particular, example radiation therapy system 900 may include C-arm gantry 910, therapeutic radiation source in the form of LINAC 921, EPID (not shown for simplicity) and treatment couch 931 to support a patient during treatment or phantom during calibration. Similar to the example in FIG. 1, example radiation therapy system 900 may be configured with an imaging system that includes at least one imaging source 941 and imager 942.

Similar to the examples in FIGs. 1-3, radiation therapy system 900 may include couch positioning assembly 930 to adjustably position treatment couch 131 supporting any suitable degrees of freedom (e.g., 3DoF, 4DoF or 6DoF). Couch positioning assembly 930 may include any suitable motors and joints (not shown for simplicity) to support linear movements and/or rotational movements. Couch positioning assembly 130 may be controlled using controller 160 in FIG. 1 using any suitable commands and control signals (see 161 in FIG. 1).

According to examples of the present disclosure, kinematic calibration may be performed to calibrate treatment couch 931 of radiation therapy system 900. Through kinematic calibration, a kinematic model associated with treatment couch 131 may be updated programmatically. Similarly, first computer system 180 may be configured to perform calibration dataset generation 401 according to block 405 in FIG. 4. First computer system 180 may include any suitable hardware and/or software components, such as interface 181 to interact with controller 160 and/or second computer system 190, and dataset generator 182 to perform block 405. Second computer system 190 (i.e., kinematic calibration system) may be configured to perform couch calibration 402 according to blocks 410-450 in FIG. 4. Second computer system 190 may include any suitable hardware and/or software components, such as interface 191 to interact with controller 160 and/or first computer system 180, kinematic calibration controller 192 to perform blocks 450-495. Various examples that have been described using FIGs. 1-8 are also applicable here and not repeated for brevity.

Computer system

The above examples can be implemented by hardware (including hardware logic circuitry), software or firmware or a combination thereof. The above examples may be implemented by any suitable computing device, computer system, etc. The computer system may include processor(s), memory unit(s) and physical NIC(s) that may communicate with each other via a communication bus, etc. The computer system may include a non-transitory computer-readable medium having stored thereon instructions or program code that, when executed by the processor, cause the processor to perform processes described herein with reference to the drawings. The term "program code" may refer generally to any suitable computer-executable instructions (e.g., scripts, programming language, machine language, etc.) in compiled and/or uncompiled form.

The techniques introduced above can be implemented in special-purpose hardwired circuitry, in software and/or firmware in conjunction with programmable circuitry, or in a combination thereof. Special-purpose hardwired circuitry may be in the form of, for example, one or more application-specific integrated circuits (ASICs), programmable logic devices (PLDs), field-programmable gate arrays (FPGAs), and others. The term 'processor' is to be interpreted broadly to include a processing unit, ASIC, logic unit, or programmable gate array etc.

The foregoing detailed description has set forth various embodiments of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or any combination thereof.

Those skilled in the art will recognize that some aspects of the embodiments disclosed herein, in whole or in part, can be equivalently implemented in integrated circuits, as one or more computer programs running on one or more computers (e.g., as one or more programs running on one or more computing systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure.

Software to implement the techniques introduced here may be stored on a non-transitory computer-readable storage medium and may be executed by one or more general-purpose or special-purpose programmable microprocessors. A "computer-readable storage medium", as the term is used herein, includes any mechanism that provides (i.e., stores and/or transmits) information in a form accessible by a machine (e.g., a computer, network device, personal digital assistant (PDA), mobile device, manufacturing tool, any device with a set of one or more processors, etc.). A computer-readable storage medium may include recordable/non recordable media (e.g., read-only memory (ROM), random access memory (RAM), magnetic disk or optical storage media, flash memory devices, etc.).

The drawings are only illustrations of an example, wherein the units or procedure shown in the drawings are not necessarily essential for implementing the present disclosure. Those skilled in the art will understand that the units in the device in the examples can be arranged in the device in the examples as described or can be alternatively located in one or more devices different from that in the examples. The units in the examples described can be combined into one module or further divided into a plurality of sub-units.

**Claims**

1. A method for a computer system to perform kinematic calibration of a treatment couch for radiation therapy, wherein the method comprises:

   obtaining a dataset of multiple calibration poses;
   generating and sending one or more first instructions to cause the treatment couch to move towards each of the multiple calibration poses in the dataset, wherein the treatment couch is associated with a kinematic model;
   determining multiple measured poses reached by the treatment couch, wherein the multiple measured poses are associated with the respective multiple calibration poses;
   determining geometric correction data for updating the kinematic model based on the multiple measured poses; and
   generating and sending one or more second instructions to update the kinematic model associated with the treatment couch based on the geometric correction data.

2. The method of claim 1, wherein determining the geometric correction data comprises:
   determining the geometric correction data by solving an optimization problem to minimize or reduce deviation data between (a) the multiple measured poses and (b) respective multiple modelled poses.

3. The method of claim 1 or claim 2, wherein determining the geometric correction data comprises:
   determining a particular modelled pose based on a forward kinematic model associated with the treatment couch and joint parameter data that is determined using an inverse kinematic model.

4. The method of any preceding claim, wherein generating and sending the one or more second instructions comprises: generating and sending the one or more second instructions to a controller to update a forward kinematic model associated with the treatment couch, wherein the forward kinematic model is for mapping joint parameter data to a particular pose based on the geometric correction data and mechanical parameter data associated with the treatment couch;
and/or:
wherein generating and sending the one or more second instructions comprises:
generating and sending the one or more second instructions to a controller to update an inverse kinematic model associated with the treatment couch, wherein the inverse kinematic model is for mapping a particular pose to joint parameter data based on the geometric correction data and mechanical parameter data associated with the treatment couch.

5. The method of any preceding claim, wherein generating and sending the one or more first instructions comprises: generating and sending the one or more first instructions to a controller to cause the treatment couch to move towards a particular calibration pose based on joint parameter data, wherein the controller is communicatively coupled with a couch positioning assembly of the treatment couch, and the joint parameter data is determined by the computer system, or a controller based on an inverse kinematic model associated with the treatment couch;
and/or:
wherein determining the multiple measured poses comprises:

   obtaining (a) imaging data from one or more imagers and (b) tracking data from one or more laser trackers; and determining the multiple measured poses based on the imaging data or the tracking data, or both.

6. A computer system, comprising:

   a processor; and
   a non-transitory computer-readable medium having stored thereon program code that, when executed by the processor, causes the processor to perform the following:

      generate and send one or more first instructions to cause the treatment couch to move towards each of multiple calibration poses, wherein the treatment couch is associated with a kinematic model;
      determine multiple measured poses reached by the treatment couch, wherein the multiple measured poses are associated with the respective multiple calibration poses;
      determine geometric correction data for updating the kinematic model based on the multiple measured poses; and
      generate and send one or more second instructions to update the kinematic model associated with the treatment couch based on the geometric correction data.

7. The computer system of claim 6, wherein the program code for determining the geometric correction data causes the processor to:
determine the geometric correction data by solving an optimization problem to minimize deviation data between (a) the multiple measured poses and (b) respective multiple modelled poses.

8. The computer system of claim 6 or claim 7, wherein the program code for determining the geometric correction data causes the processor to:
determine a particular modelled pose based on a forward kinematic model associated with the treatment couch and joint parameter data that is determined using an inverse kinematic model.

9. The computer system of any of claim 6 to claim 8, wherein the program code for generating and sending the one or more second instructions causes the processor to:
generate and send the one or more second instructions to a controller to update a forward kinematic model associated with the treatment couch, wherein the forward kinematic model is for mapping joint parameter data to a particular pose based on the geometric correction data and mechanical parameter data associated with the treatment couch;
and/or:
wherein the program code for generating and sending the one or more second instructions causes the processor to:
generate and send the one or more second instructions to a controller to update an inverse kinematic model associated with the treatment couch, wherein the inverse kinematic model is for mapping a particular pose to joint parameter data based on the geometric correction data and mechanical parameter data associated with the treatment couch.

10. The computer system of any of claim 6 to claim 9, wherein the program code for generating and sending the one or more first instructions causes the processor to:

generate and send the one or more first instructions to a controller to causes the treatment couch to move towards a particular calibration pose based on joint parameter data, wherein the controller is communicatively coupled with a couch positioning assembly of the treatment couch, and the joint parameter data is determined by the computer system, or a controller based on an inverse kinematic model associated with the treatment couch; 'and/or:

wherein the program code for determining the multiple measured poses causes the processor to:

obtain (a) imaging data from one or more imagers and (b) tracking data from one or more laser trackers; and determine the multiple measured poses based on the imaging data or the tracking data, or both.

11. A radiation therapy system, comprising:

a treatment couch that is associated with a kinematic model and requires calibration; and a computer system to:

generate and send one or more first instructions to cause the treatment couch to move towards each of multiple calibration poses, wherein the treatment couch is associated with a kinematic model; determine multiple measured poses reached by the treatment couch, wherein the multiple measured poses are associated with the respective multiple calibration poses; determine geometric correction data for updating the kinematic model based on the multiple measured poses; and generate and send one or more second instructions to update the kinematic model associated with the treatment couch based on the geometric correction data.

12. The radiation therapy system of claim 11, wherein the computer system is to determine the geometric correction data by performing the following: determine the geometric correction data by solving an optimization problem to minimize deviation data between (a) the multiple measured poses and (b) respective multiple modelled poses.

13. The radiation therapy system of claim 11 or claim 12, wherein the computer system is to determine the geometric correction data by performing the following: determine a particular modelled pose from the multiple modelled poses based on a forward kinematic model and joint parameter data that is determined using an inverse kinematic model.

14. The radiation therapy system of any of claim 11 to claim 13, wherein the computer system is to generate and send the one or more second instructions by performing the following: generate and send the one or more second instructions to a controller to update a forward kinematic model associated with the treatment couch, wherein the forward kinematic model is for mapping joint parameter data to a particular pose based on the geometric correction data and mechanical parameter data associated with the treatment couch; and/or:

wherein the computer system is to generate and send the one or more second instructions by performing the following: generate and send the one or more second instructions to a controller to update an inverse kinematic model associated with the treatment couch, wherein the inverse kinematic model is for mapping a particular pose to joint parameter data based on the geometric correction data and mechanical parameter data associated with the treatment couch.

15. The radiation therapy system of any of claim 11 to claim 14, wherein the computer system is to generate and send the one or more first instructions by performing the following: generate and send the one or more first instructions to a controller to cause the treatment couch to move towards a particular calibration pose based on joint parameter data, wherein the controller is communicatively coupled with a couch positioning assembly of the treatment couch, and the joint parameter data is determined by the computer system, or a controller based on an inverse kinematic model associated with the treatment couch; and/or:

wherein the computer system is to determine the multiple measured poses by performing the following:

obtain (a) imaging data from one or more imagers and (b) tracking data from one or more laser trackers; and determine the multiple measured poses based on the imaging data or the tracking data, or both.

**100**

Treatment delivery
machine **110**

LINAC
**120**

**111**

**112**

Treatment
beam (MV)
**123**

Patient
**132** **150**

Imaging
System
(kV)
**140**

Source **141**

**143**
Imaging
beam
(kV)

Imager **142**

**131**

Couch Positioning
Assembly **130**

EPID **121**

Control
Signal(s)
**161**

Projection
Image Data
(MV) **170**

Projection
Image Data
(kV) **171**

Controller
**160**

Interface(s)
**181**

Dataset Generator
**182**

First Computer System
**180**

Interface(s)
**191**

Kinematic Calibration
Controller **192**

Kinematic Calibration System
(Second Computer System)
**190**

**FIG. 1**

200

110

210

111

121

112

141

204

203

Z

Y 131

X

130

210

120

142

FIG. 2

300

131    303

312

304

304

X θ$_X$
Y θ$_Y$

Z θ$_Z$

311

302

130

301

301    305

Degrees of Freedom (DoF)*

| X | Lateral | 321 |
|---|---------|-----|
| Y | Longitudinal | 322 |
| Z | Vertical | 323 |
| θ$_X$ | Pitch | 324 |
| θ$_Y$ | Roll | 325 |
| θ$_Z$ | Yaw | 326 |

* Examples: 3DoF, 4DoF, 6DoF, etc.

FIG. 3

400

**401 DATASET GENERATION**
*E.g., Computer System 180*

**405**
Generate/select dataset of calibration poses, where
SET={POSE-n} for n=1,...,N

POSE-1
POSE-2
:
POSE-N

**402 KINEMATIC CALIBRATION**
*E.g., Computer System 190*

**410**
Obtain SET={POSE-n} for couch associated with kinematic model (KM)

For each n

**420**
Generate and send first instructions to cause couch to move towards POSE-n

**421**
Determine joint parameter data (JOINT-n) associated with POSE-n using inverse kinematic model

**430**
Determine measured pose (POSE'-n) reached by couch

**431**
Determine POSE'-n based on imaging data from imager(s) and/or tracking data from laser tracker(s)

n<N

n=N

**440**
Determine geometric parameter data (p) based on {POSE'-n}

**441**
Determine p = geometric parameter data by solving optimization problem

**450**
Generate and send second instructions to update KM based on geometric parameter data (p)

**FIG. 4**

**500**

Phantom
**501**

Isocenter
**502**

**303**

**131**

$J_{\theta X}$

$c_1 = L(\text{Roll} \rightarrow$ Table Base Point)
**551\***

$J_Y$ $J_{\theta Y}$

$c_2 = H(\text{IEC} \rightarrow \text{Lat})$
**552\***

$J_Z$

**302**

$J_{\theta Z}$

$c_3 = L(\text{IEC} \rightarrow \text{Lat})$
**553\***

$J_X$ **301**

\* **550** Couch-dependent mechanical parameter data
$c = [c_1, c_2, c_3, \ldots]$

**510**
Joint parameter data
(JOINT)

| $J_X$ |
| :---: |
| $J_Y$ |
| $J_Z$ |
| $J_{\theta X}$ |
| $J_{\theta Y}$ |
| $J_{\theta Z}$ |

*Couch joint space*

**530**
Forward Kinematic Model
JOINT=FKM(POSE,c,p)\*\*

**540**
Inverse Kinematic Model
POSE=IKM(JOINT,c,p)\*\*

**520**
Pose data
(POSE)

| $X$ |
| :---: |
| $Y$ |
| $Z$ |
| $\theta_X$ |
| $\theta_Y$ |
| $\theta_Z$ |

*IEC fixed coordinate system*

\*\*Geometric correction data
$p = [p_1, p_2, p_3, \ldots]$

**FIG. 5**

600

Laser tracker(s)
**601**

120

111

112

141

Phantom
610

1

142

131

130

110

121

IMAGING
DATA
**640**

4

TRACKING
DATA
**641**

4

MOVETO
(JOINT-n)
**635**

3

Interface(s) **191**

Controller
**160**

JOINT=IKM(POSE,c)
POSE=FKM(JOINT,c)
**530/540**

MOVETO
(POSE-n)
**630**

3

Kinematic Calibration
Controller **192**

Joint Parameter Data **620**

JOINT-1=IKM(POSE-1,c)
⋮
JOINT-N=IKM(POSE-N,c)

2

*Kinematic
Calibration*
690

JOINT=IKM(POSE,c,p)
POSE=FKM(JOINT,c,p)
**680**

8

Measured Pose Data **650**

n=1: POSE'-1
⋮
N=1: POSE'-N

5

Geometric Correction Data **660**

$$\arg\min_{p} f(p)$$

f(p)=diff(POSE'-n,
MODEL-n(p))
MODEL-n(p)=FKM(JOINT-n,c,p)

6

7

UPDATE(p)
**670**

Couch Calibration System
**190**

**FIG. 6**

700

Body
701

Phantom
610

Holder
703

Markers
702

Imaging data acquisition
710

MV/kV Imaging Data **640**

Measured pose estimation
720

POSE' -n
650

| $X'$ |
| $Y'$ |
| $Z'$ |
| $\theta'_X$ |
| $\theta'_Y$ |
| $\theta'_Z$ |

*E.g., 6DoF pose*

**FIG. 7**

800

Geometric correction data **660**

| From | To | Components | Example | |
|---|---|---|---|---|
| Isocenter | Lateral joint | $X, Y, Z,$ $\theta_Y, \theta_Z$ | 0.5 mm 0.2 deg | 810 |
| Yaw joint | Vertical joint | $Y$ | 0.5 mm | 820 |
| Pitch joint | Roll joint | $Z$ | 0.4 mm | 830 |
| Roll joint | Table top base point | $X, Y, Z,$ $\theta_Z$ | 0.5 mm 0.2 deg | 840 |
| Vertical joint | Longitudinal joint | $\theta_X, \theta_Y, \theta_Z$ | 0.1 deg | 850 |

**FIG. 8**

900

**FIG. 9**

# EUROPEAN SEARCH REPORT

**Application Number**

EP 25 19 6776

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2019/056134 A1 (UNIV DALHOUSIE [CA]) 28 March 2019 (2019-03-28) * the whole document * ----- | 1-15 | INV. A61N5/10 A61B6/58 |
| A | US 2008/235970 A1 (CRAMPTON STEPHEN JAMES [GB]) 2 October 2008 (2008-10-02) * paragraph [0420] * ----- | 1-15 | |

**TECHNICAL FIELDS SEARCHED (IPC)**

A61N
A61B

The present search report has been drawn up for all claims

| Place of search | Date of completion of the search | Examiner |
|---|---|---|
| Munich | 11 December 2025 | Beck, Ewa |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.........................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 19 6776

This annex lists the patent family members relating to the patent documents cited in  the above-mentioned European search report.
The members are as contained in the European Patent Office EDP file on
The European Patent Office is in no way liable for these particulars which are merely given for the purpose of information.

11-12-2025

| Patent document cited in search report | Publication date | Patent family member(s) | Publication date |
|---|---|---|---|
| WO 2019056134 A1 | 28-03-2019 | NONE | |
| US 2008235970 A1 | 02-10-2008 | CA        2522097 A1 | 11-11-2004 |
| | | EP        1633534 A1 | 15-03-2006 |
| | | GB        2417090 A | 15-02-2006 |
| | | KR    20060015557 A | 17-02-2006 |
| | | US     2005166413 A1 | 04-08-2005 |
| | | US     2008235970 A1 | 02-10-2008 |
| | | WO     2004096502 A1 | 11-11-2004 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 18809563 B

**Non-patent literature cited in the description**

- **W. H. PRESS** ; **S. H. TEUKOLSKY** ; **W. T. VETTERLING** ; **B. P. FLANNERY**. *Numerical Recipes: The Art of Scientific Computing*